# EUROPEAN PATENT SPECIFICATION

(11) **EP 0 647 227 B1**
(45) Date of publication and mention of the grant of the patent: **09.10.1996**
(21) Application number: 93914675.9
(22) Date of filing: 18.06.1993
(51) Int. Cl.: C07D 473/30, A61K 31/52

(54) **PURINONE ANTIANGINAL AGENTS**
ANTIANGINALE PURINONE
PURINONES UTILISES COMME ANTIANGINEUX

(30) Priority: 26.06.1992 GB 9213623
(43) Date of publication of application: 12.04.1995
(73) Proprietor: Pfizer Limited, Sandwich Kent CT13 9NJ (GB); Pfizer Research and Development Company, N.V./S.A., Dublin 2 (IE)
(72) Inventor: TERRETT, Nicholas, Kenneth, Sandwich Kent CT13 9NJ (GB)
(74) Representative: Moore, James William, Dr.
(86) International application number: EP9301561
(87) International publication number: WO9400453

(56) References cited:
- EP-A- 0 293 063
- EP-A- 0 347 146
- EP-A- 0 352 960
- GB-A- 1 338 235
- US-A- 5 073 559

## Description

This invention relates to a series of purin-6-ones, which are potent and selective inhibitors of cyclic guanosine 3',5'-monophosphate phosphodiesterase (cGMP PDE), having utility in a variety of therapeutic areas including the treatment of cardiovascular disorders such as angina, hypertension, heart failure and atherosclerosis.

The compounds of the invention exhibit selectivity for inhibition of cGMP PDEs rather than cyclic adenosine 3',5'-monophosphate phosphodiesterases (cAMP PDEs) and, as a consequence of this selective PDE inhibition, cGMP levels are elevated, which in turn can give rise to beneficial anti-platelet, anti-neutrophil, anti-vasospastic and vasodilatory activity, as well as potentiation of the effects of endothelium-derived relaxing factor (EDRF) and nitrovasodilators. Thus the compounds have utility in the treatment of a number of disorders, including stable, unstable and variant (Prinzmetal) angina, hypertension, pulmonary hypertension, congestive heart failure, atherosclerosis, conditions of reduced blood vessel patency e.g. post-percutaneous transluminal coronary angioplasty (post-PTCA), peripheral vascular disease, stroke, bronchitis, allergic asthma, chronic asthma, allergic rhinitis, glaucoma, and diseases characterised by disorders of gut motility, e.g. irritable bowel syndrome (IBS).

Australian patent application AU-A-10956/88 and European patent application EP-A-0352960 disclose certain purin-6-ones which, unlike the compounds of the present invention, are N-unsubstituted in the imidazole portion of the purinone bicyclic system. These prior art purinones are reported to be selective cGMP PDE inhibitors with bronchodilator and vasodilator activity, of value in combatting asthma, bronchitis, angina, hypertension and congestive heart failure. However, compared with the 9-alkylpurin-6-ones of the present invention, they are not particularly potent cGMP PDE inhibitors.

The compounds of the present invention have the formula (I): 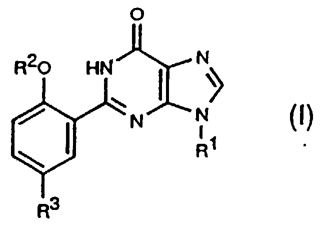 and pharmaceutically acceptable salts thereof,
wherein
R¹ is C₁-C₄ alkyl;
R² is C₂-C₄ alkyl;
R³ is H or SO₂NR⁴R⁵;
R⁴ and R⁵ together with the nitrogen atom to which they are attached form a pyrrolidino, piperidino, morpholino or 4-N-(R⁶)-1-piperazinyl group;
and
R⁶ is H or C₁-C₃ alkyl.

In the above definition, unless otherwise indicated, alkyl groups having three or more carbon atoms may be straight chain or branched chain.

The compounds of formula (I) may contain one or more asymmetric centres and thus they can exist as stereoisomers, i.e. as enantiomers or as diastereoisomers. The invention includes both mixtures thereof and the separated individual stereoisomers.

The compounds of formula (I) may also exist in tautomeric forms and the invention includes both mixtures thereof and the separated individual tautomers.

Also included in the invention are radiolabelled derivatives of compounds of formula (I) which are suitable for biological studies.

The pharmaceutically acceptable salts of the compounds of formula (I) are, for example, non-toxic acid addition salts formed with inorganic acids such as hydrochloric, hydrobromic, sulphuric and phosphoric acid, with organo-carboxylic acids, or with organosulphonic acids. Compounds of formula (I) can also provide pharmaceutically acceptable metal salts, in particular non-toxic alkali metal salts, with bases. Examples include the sodium and potassium salts. For a review on suitable pharmaceutical salts, see J. Pharm, Sci., 1977, 66, 1.

A preferred group of compounds of formula (I) is that wherein R¹ and R² are each independently ethyl or n-propyl; R⁴ and R⁵ together with the nitrogen atom to which they are attached form a 4-N-(R6)-1-piperazinyl group; and R³ and R⁶ are as previously defined.

A particularly preferred group of compounds of formula (I) is that wherein R¹ is n-propyl; R² is ethyl; and R³ is 1-piperazinylsulphonyl or 4-methyl-1-piperazinylsulphonyl.

In another aspect, the present invention provides processes for the preparation of compounds of formula (I) and pharmaceutically acceptable salts thereof, as hereinafter described.
(A) A compound of formula (I), wherein R³ is SO₂NR⁴R⁵, may be obtained from a compound of formula (I) wherein R³ is H, and R² and R³ are as previously defined for formula (I). This can be achieved via the intermediacy of a sulphonyl halide of formula (II): 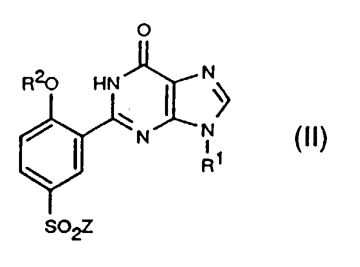 wherein Z is halo, preferably chloro, and R¹ and R² are as previously defined for formula (I), by reaction with an amine of formula (III):

   HNR⁴R⁵ (III)

   wherein R⁴ and R⁵ are as previously defined for formula (I). The reaction is generally carried out at ambient temperature, preferably in the presence of a solvent, e.g. a C₁-C₃ alkanol, using about a 5-fold excess of (III) to scavenge the acid by-product (HZ) and, in the case of piperazine (R⁶ is H), to minimise bissulphonamide formation.
   A compound of formula (II) is obtainable by the application of known methods for the introduction of a SO₂Z group into a benzene ring; for example, when Z is chloro, by the action of excess chlorosulphonic acid at from about 0°C to about ambient temperature.
   A compound of formula (I) wherein R³ is H, R¹ and R² are as previously defined for formula (I), may be obtained from a compound of formula (IV): 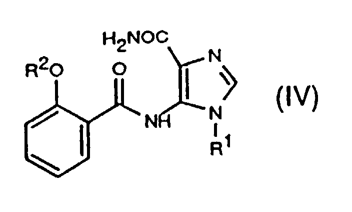 wherein R¹ and R² are as previously defined for formula (I), by the application of known cyclisation methods for pyrimidinone ring formation. Thus, for example, cyclisation may be effected by the treatment of (IV) with a base such as sodium hydroxide or potassium carbonate, optionally in the presence of excess hydrogen peroxide, in an ethanol-water medium, at about the reflux temperature of the reaction medium.
   Alternatively, cyclisation may be effected with polyphosphoric acid at about 140°C.
   A compound of formula (IV) may be prepared from a compound of formula (V): 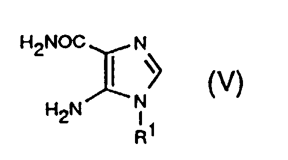 wherein R¹ is as previously defined for formula (IV), by reaction with an acyl halide of formula (VI): 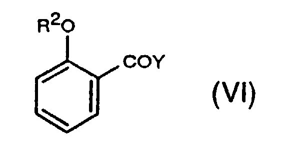 wherein Y is halo, preferably chloro or bromo, and R² is as previously defined for formula (IV). The reaction is generally carried out using from about 1 to about 2 equivalents of (VI) in the presence of an excess of a tertiary amine such as triethylamine or pyridine to act as scavenger for the acid by-product (HY), optionally in the presence of a catalyst such as 4-dimethylaminopyridine, in an inert solvent such as dichloromethane, at from about 0°C to ambient temperature for 2-72 hours. For convenience, pyridine may also be used as solvent.
   Alternative methods of converting (V) to a compound of formula (I) wherein R³ is H, and R² and R³ are as previously defined for formula (I), will be evident to persons skilled in the art. For example, a one-step procedure involves the reaction of (V) with an amidine of formula (VII): 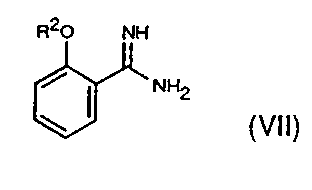 wherein R² is as previously defined for formula (I), at elevated temperature. The aminoimidazolecarboxamide component may be in the form of an acid addition salt, e.g. the hydrochloride, and the reaction may be conducted in the absence of solvent, or in a suitable solvent such as a C₁-C₄ alcohol, pyridine or N-methylpyrrolidone, at from about 65°C to about 200°C.
   A compound of formula (V) may be prepared from an amine of formula R¹NH₂ (VIII), wherein R¹ is as previously defined for formula (V), and 2-amino-2-cyanoacetamide in a two-stage process, according to the procedure disclosed in EP-A-66,909. Firstly, the latter component is condensed with a tri-lower alkyl orthoformate, e.g. trimethyl or triethylorthoformate, in a suitable solvent such as acetonitrile, at the reflux temperature of the reaction medium, then the intermediate formimidate is treated in situ with (VIII) at about ambient temperature. 2-Amino-2-cyanoacetamide can be obtained by dithionite reduction of ethyl 2-cyano-2-(hydroxyimino)acetate, followed by in situ amination of the intermediate ethyl 2-amino-2-cyanoacetate, as reported in Chem. and Ind., 1980, (13), 541.
(B) A compound of formula (I), wherein R³ is SO₂NR⁴R⁵, may be obtained more directly from a compound of formula (V) by employing an acyl halide of formula (IX) or an amidine of formula (X): 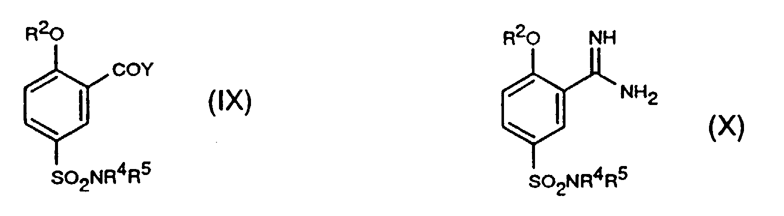 wherein R², R⁴ and R⁵ are as previously defined for formula (I) and Y is as previously defined for formula (VI), in reactions analogous to those described above for (VI) and (VII) respectively.
(C) A compound of formula (I) may also be obtained from the corresponding phenol precursor, i.e. a compound of formula (I) wherein R² is H and R¹ and R³ are as previously defined for formula (I), thereby introducing R² at the final stage of the synthesis. This may be achieved by selective O-alkylation of the phenolic group under standard conditions, using the appropriate C₂-C₄ alkyl bromide, iodide or sulphonate, in the presence of a base such as anhydrous potassium carbonate, in a suitable solvent, e.g. 2-butanone, at from about ambient temperature to about the reflux temperature of the reaction medium. Alternatively, the alkylation may be effected under typical Mitsunobu reaction conditions. A variety of other selective O-alkylation procedures will be obvious to persons skilled in the art.
(D) Certain piperazines of formula (I), wherein R⁶ is as previously defined for formula (I) but is not hydrogen, may be prepared directly from the corresponding 4-N-unsubstituted piperazine analogue, i.e. the precursor wherein R⁶ is H, by standard alkylation procedures such as use of the appropriate C₁-C₃ alkyl bromide, iodide or sulphonate, in the presence of a base such as anhydrous potassium carbonate, in a suitable solvent, e.g. 2-butanone, at from about ambient temperature to about the reflux temperature of the reaction medium.
   Compounds of formulae (VI), (VII), (IX) and (X), and the various reagents required for the processes hereinbefore disclosed, when neither commercially available nor subsequently described, can be obtained by conventional synthetic procedures, in accordance with standard textbooks on organic chemistry or literature precedent, from readily accessible starting materials using appropriate reagents and reaction conditions.
   Moreover, persons skilled in the art will be aware of variations of, and alternatives to, those processes described hereinafter in the the Examples and Preparations sections such that all the compounds defined by formula (I) are obtainable.
   The pharmaceutically acceptable acid addition salts of the compounds of formula (I) which contain a basic centre may also be prepared in a conventional manner. For example a solution of the free base is treated with the appropriate acid, either neat or in a suitable solvent, and the resulting salt isolated either by filtration or by evaporation under vacuum of the reaction solvent. Pharmaceutically acceptable base addition salts can be obtained in an analogous manner by treating a solution of a compound of formula (I) with the appropriate base. Both types of salt may be formed or interconverted using ion-exchange resin techniques.
   The biological activities of the compounds of the present invention were determined by the following test methods.

### Phosphodiesterase activity

Compound affinities for cGMP and cAMP PDEs are assessed by determination of their IC₅₀ values (the concentration of inhibitor required for 50% inhibition of enzyme activity). The PDE enzymes are isolated from rabbit platelets and rat kidney, essentially by the method of W.J. Thompson et al. (Biochem., 1971, 10, 311). The calcium/calmodulin (Ca/CAM)-independent cGMP PDE and the cGMP-inhibited cAMP PDE enzymes are obtained from rabbit platelets whilst, of the four major PDE enzymes of the rat kidney, the Ca/CAM-dependent cGMP PDE (fraction I) is isolated. Assays are performed using a modification of the "batch" method of W.J. Thompson and M.M. Appleman (Biochem., 1979, 18, 5228). Results from these tests show that the compounds of the present invention are potent and selective inhibitors of both cGMP PDEs.

### Platelet anti-aggregatory activity

This is assessed by the determination of a compound's ability to inhibit platelet aggregation in vitro induced by platelet activating factor (PAF), and to potentiate the platelet antiaggregatory action in vitro of activators of guanylate cyclase such as nitroprusside and EDRF. Washed platelets are prepared essentially by the method of J.F. Mustard et al. (Methods in Enzymol., 1989, 169, 3) and aggregation is determined using standard turbidimetric techniques as described by G.V.R. Born, (J. Physiol. (Lond), 1962, 162, 67P).

### Antihypertensive activity

This is assessed following intravenous or oral administration of a compound to spontaneously hypertensive rats. Blood pressure is recorded via a cannula implanted in the carotid artery of either conscious or anaesthetised animals.

For administration to man in the curative or prophylactic treatment of angina, hypertension or congestive heart failure, oral dosages of the compounds will generally be in the range of from 4-800 mg daily for an average adult patient (70 kg). Thus for a typical adult patient, individual tablets or capsules contain from 2-400 mg of active compound, in a suitable pharmaceutically acceptable vehicle or carrier, for administration in single or multiple doses, once or several times per day. Dosages for intravenous, buccal or sublingual administration will typically be within the range of from 1-400 mg per single dose as required. In practice the physician will determine the actual dosing regimen which will be most suitable for an individual patient and it will vary with the age, weight and response of the particular patient. The above dosages are exemplary of the average case but there can be individual instances in which higher or lower dosage ranges may be merited, and such are within the scope of this invention.

For human use, the compounds of formula (I) can be administered alone, but will generally be administered in admixture with a pharmaceutical carrier selected with regard to the intended route of administration and standard pharmaceutical practice. For example, they may be administered orally, buccally or sublingually, in the form of tablets containing excipients such as starch or lactose, or in capsules or ovules either alone or in admixture with excipients, or in the form of elixirs or suspensions containing flavouring or colouring agents. The compounds may also be injected parenterally, for example intravenously, intramuscularly, subcutaneously or intracoronarily. For parenteral administration, they are best used in the form of a sterile aqueous solution which may contain other substances, for example salts, or monosaccharides such as mannitol or glucose, to make the solution isotonic with blood.

Thus the invention provides a pharmaceutical composition comprising a compound of formula (I), or a pharmaceutically acceptable salt thereof, together with a pharmaceutically acceptable diluent or carrier.

The invention also provides a compound of formula (I), or a pharmaceutically acceptable salt thereof, or a pharmaceutical composition containing either entity, for use in medicine.

The invention further provides the use of a compound of formula (I), or a pharmaceutically acceptable salt thereof, or a pharmaceutical composition containing either entity, for the manufacture of a medicament for the treatment of stable, unstable and variant (Prinzmetal) angina, hypertension, pulmonary hypertension, congestive heart failure, atherosclerosis, stroke, peripheral vascular disease, conditions of reduced blood vessel patency, chronic asthma, bronchitis, allergic asthma, allergic rhinitis, glaucoma or diseases characterised by disorders of gut motility.

In a further aspect, the invention provides a method of treating or preventing stable, unstable and variant (Prinzmetal) angina, hypertension, pulmonary hypertension, congestive heart failure, atherosclerosis, stroke, peripheral vascular disease, conditions of reduced blood vessel patency, chronic asthma, bronchitis, allergic asthma, allergic rhinitis, glaucoma or diseases characterised by disorders of gut motility, in a mammal (including a human being), which comprises administering to said mammal a therapeutically effective amount of a compound of formula (I), or a pharmaceutically acceptable salt thereof, or a pharmaceutical composition containing either entity.

The invention also includes any novel intermediates of formulae (II) and (IV) disclosed herein.

The syntheses of the compounds of the invention and of the intermediates for use therein are illustrated by the following Examples and Preparations. The purity of the compounds was routinely monitored by thin layer chromatography (TLC) using Merck Kieselgel 60 F₂₅₄ plates. ¹H-Nuclear magnetic resonance (NMR) spectra were recorded using either a Nicolet QE-300 or a Bruker AC-300 spectrometer and were in all cases consistent with the proposed structures.

Ambient temperature means 20-25°C.

### EXAMPLE 1

### 2-(2-Ethoxyphenyl)-9-n-propylpurin-6-one

A solution of 5-(2-ethoxybenzamido)-l-n-propylimidazole-4-carboxamide (Preparation 3; 0.35 g, 0.0011 mol) in ethanol (8 ml) was added to a stirred mixture of aqueous hydrogen peroxide (30% solution; 0.3 ml, 0.0026 mol), water (15 ml) and sodium hydroxide (0.2 g, 0.005 mol). The resulting mixture was heated at 90°C for 7 hours, stirred at ambient temperature for a further 15 hours, then evaporated under vacuum. The residue was dissolved in water (20 ml), and the resulting solution acidified with hydrochloric acid to pH 4 and then extracted with dichloromethane (2 x 30 ml). The combined organic extracts were washed with water (25 ml), dried (MgSO₄) and evaporated under vacuum to give the title compound, which crystallised from ethyl acetate as a colourless solid (0.17 g, 52%), m.p. 172-174°C. Found: C,64.57; H,6.15; N,18.99. C₁₆H₁₈N₄O₂ requires C,64.41; H,6.08; N,18.78%.

### EXAMPLE 2

### 2-[2-Ethoxy-5-(1-piperazinylsulphonyl)phenyl]-9-n-propylpurin-6-one

2-(2-Ethoxyphenyl)-9-n-propylpurin-6-one (0.5 g, 0.00167 mol) was added portionwise to ice-cold chlorosulphonic acid (3 ml) and the mixture stirred at ambient temperature for 15 hours. The resulting solution was added cautiously to stirred ice/water (50 g) and the mixture extracted with dichloromethane (2 x 50 ml). The combined organic extracts were dried (MgSO₄), then evaporated under vacuum to give 2-(5-chlorosulphonyl-2-ethoxyphenyl)-9-n-propylpurin-6-one (0.63 g) which was used without further purification.

A mixture of piperazine (0.36 g, 0.0041 mol), the preceding sulphonyl chloride (0.31 g, 0.00078 mol) and ethanol (15 ml) was stirred at ambient temperature for 15 hours, then evaporated under vacuum. The residue was chromatographed twice on silica gel (12 g) using a dichloromethane:methanol:aqueous ammonia elution gradient (95:5:0 to 90:10:0 to 90:10:1) to give the title compound, which crystallised from acetonitrile as a colourless hydrated solid (0.19 g, 55%), m.p. 174-176°C. Found: C,53.22; H,6.08; N,18.56. C₂₀H₂₆N₆O₄S; 0.25 H₂O requires C,53.26; H,5.92; N,18.63%.

### EXAMPLE 3

### 2-[2-Ethoxy-5-(4-methyl-1-piperazinylsulphonyl)phenyl]-9-n-propylpurin-6-one

The title compound was prepared from 2-(2-ethoxyphenyl)-9-n-propylpurin-6-one and 1-methylpiperazine, following the procedure of Example 2, and was obtained as a colourless solid (81%), m.p. - softens at 150°C. Found: C,54.94; H,6.30; N,17.92. C₂₁H₂₈N₆O₄S requires C,54.76; H,6.13; N,18.25%.

### PREPARATION 1

### 2-Amino-2-cyanoacetamide

Ethyl 2-cyano-2-(hydroximino)acetate (30.0 g, 0.21 mol) was dissolved in a stirred mixture of saturated aqueous sodium bicarbonate solution (90 ml) and water (180 ml), and sodium dithionite (102 g, 0.59 mol) was then added portion-wise. The resulting mixture was stirred at ambient temperature for 0.5 hour, then extracted with dichloromethane (4 x 200 ml). The organic extracts were combined, washed with saturated brine (200 ml), dried (Na₂SO₄) and evaporated under vacuum. The residue was stirred at ambient temperature for 1 hour with aqueous ammonia solution (SG 0.880, 250 ml) and, after cooling, the resulting solid was collected by filtration and dried under vacuum to give the title compound as a colourless solid (2.8 g, 13%), m.p. 123-125°C. Rf 0.12 (SiO2; dichloromethane: methanol:aqueous ammonia, 90:10:1).

### PREPARATION 2

### 5-Amino-1-n-propylimidazole-4-carboxamide

A mixture of 2-amino-2-cyanoacetamide (2.8 g, 0.0282 mol), trimethylorthoformate (3.4 g, 0.0321 mol) and acetonitrile (55 ml) was heated under reflux for 0.75 hour, then allowed to cool. n-Propylamine (1.8 g, 0.0305 mol) was then added dropwise, and the resulting mixture stirred at ambient temperature for 36 hours. The solid which precipitated was collected by filtration and crystallised from methanol to give the title compound as a colourless solid (2.9 g, 61%), m.p. 241-243°C. Found: C,49.67; H,7.15; N,33.64. C₇H₁₂N₄O requires C,49.98; H,7.19; N,33.31%.

### PREPARATION 3

### 5-(2-Ethoxybenzamido)-1-n-propylimidazole-4-carboxamide

Oxalyl chloride (1.74 g, 0.137 mol) was added dropwise to a stirred solution of 2-ethoxybenzoic acid (1.1 g, 0.00663 mol) and dimethylformamide (0.1 ml) in dichloromethane (20 ml) and the resulting mixture stirred at ambient temperature for 4 hours, then evaporated under vacuum. The residue was azeotroped with dichloromethane (2 x 20 ml), then added dropwise to a stirred solution of 5-amino-1-n-propylimidazole-4-carboxamide (0.9 g, 0.00536 mol) in pyridine (20 ml). The resulting solution was stirred at ambient temperature for 3 days, then evaporated under vacuum. The residue was dissolved in dichloromethane (50 ml), and the solution washed with 1N hydrochloric acid (30 ml), dried (Na₂SO₄) and evaporated under vacuum. Chromatography of the crude product on silica gel (15 g) using a methanol in dichloromethane elution gradient (2-6% methanol), followed by crystallisation from ethanol, gave the title compound as a colourless solid (1.3 g, 77%), m.p. 182-184°C. Found: C,60.85; H,6.40; N,18.06. C₁₆H₂₀N₄O₃ requires C,60.74; H,6.37 ; N,17.71%.

### Biological activity

The following Table illustrates the in vitro activities for the compounds of the invention.

**TABLE**

| IN VITRO PDE INHIBITORY DATA: SELECTIVITY BETWEEN CALCIUM/CALMODULIN (Ca/CAM)-INDEPENDENT cGMP PDE AND cGMP-INHIBITED cAMP PDE | | | |
|---|---|---|---|
| EXAMPLE | IC₅₀(nM) | | SELECTIVITY RATIO |
| | cGMP | cAMP | |
| 1 | 97 | >100,000 | >1,030 |
| 2 | 16 | 100,000 | 6,250 |
| 3 | 6.4 | 80,000 | 12,500 |

## Claims

1. A compound of formula (I): 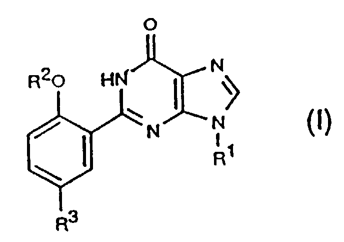 or a pharmaceutically acceptable salt thereof,
wherein
R¹ is C₁-C₄ alkyl;
R² is C₂-C₄ alkyl;
R³ is H or SO₂NR⁴R⁵;
R⁴ and R⁵ together with the nitrogen atom to which they are attached form a pyrrolidino, piperidino, morpholino or 4-N-(R⁶)-1-piperazinyl group;
and
R⁶ is H or C₁-C₃ alkyl.

2. A compound according to claim 1 wherein R¹ and R² are each independently ethyl or n-propyl; and R⁴ and R⁵ together with the nitrogen atom to which they are attached form a 4-N-(R₆)-1-piperazinyl group.

3. A compound according to claim 2 wherein R¹ is n-propyl; R² is ethyl; and R³ is 1-piperazinylsulphonyl or 4-methyl-l-piperazinylsulphonyl.

4. A pharmaceutical composition comprising a compound of formula (I), or a pharmaceutically acceptable salt thereof, according to any one of claims 1 to 3, together with a pharmaceutically acceptable diluent or carrier.

5. A compound of formula (I), or a pharmaceutically acceptable salt thereof, or a pharmaceutical composition containing either entity, according to any one of claims 1 to 4, for use in medicine.

6. The use of a compound of formula (I), or a pharmaceutically acceptable salt thereof, or a pharmaceutical composition containing either entity, according to any one of claims 1 to 4, for the manufacture of a medicament for the treatment of stable, unstable and variant (Prinzmetal) angina, hypertension, pulmonary hypertension, congestive heart failure, atherosclerosis, stroke, peripheral vascular disease, conditions of reduced blood vessel patency, chronic asthma, bronchitis, allergic asthma, allergic rhinitis, glaucoma or diseases characterised by disorders of gut motility.

7. A process for the preparation of a compound of formula (I): 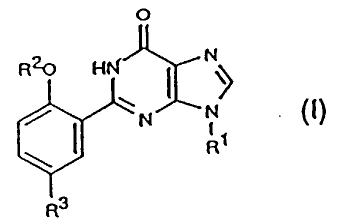 or a pharmaceutically acceptable salt thereof,
wherein
R¹ is C₁-C₄ alkyl;
R² is C₂-C₄ alkyl;
R³ is SO₂NR⁴R⁵;
R⁴ and R⁵ together with the nitrogen atom to which they are attached form a pyrrolidino, piperidino, morpholino or 4-N-(R⁶)-1-piperazinyl group;
and
R⁶ is H or C₁-C₃ alkyl;
which comprises reacting a compound of formula (II): 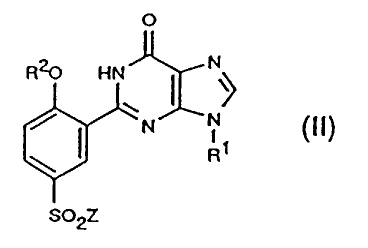 wherein Z is halo, and R¹ and R² are as previously in this claim, with a compound of formula (III):
HNR⁴R⁵ (III)
wherein R⁴ and R⁵ are as previously defined in this claim, and optionally converting the required product to a pharmaceutically acceptable salt thereof.

8. A process according to claim 7 wherein a 5-fold excess of a compound of formula (III) is employed together with a C₁-C₃ alkanol as solvent.

9. A process for the preparation of a compound of formula (I): 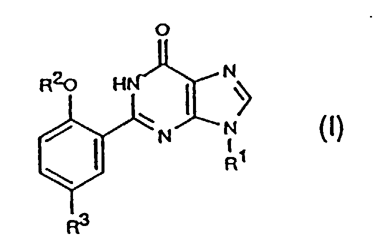 or a pharmaceutically acceptable salt thereof,
wherein
R¹ is C₁-C₄ alkyl;
R² is C₂-C₄ alkyl;
and
R³ is H;
which comprises cyclisation of a compound of formula (IV) : 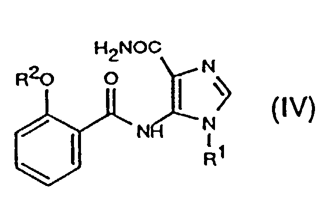 wherein R¹ and R² are as previously defined in this claim.

10. A process according to claim 9 wherein the cyclisation is achieved using a base, optionally in the presence of excess hydrogen peroxide, in aqueous ethanol.

11. A process according to claim 10 wherein the base is sodium hydroxide or potassium carbonate.

12. A process according to claim 7 or claim 8 wherein R¹ and R² are each independently ethyl or n-propyl; and R⁴ and R⁵ together with the nitrogen atom to which they are attached form a 4-N-(R⁶)-1-piperazinyl group.

13. A process according to claim 12 wherein R¹ is n-propyl; R² is ethyl; and R³ is 1-piperazinylsulphonyl or 4-methyl-l-piperazinylsulphonyl.

14. A process according to any one of claims 9 to 11 wherein R¹ and R² are each independently ethyl or n-propyl.

15. A process according to claim 14 wherein R¹ is n-propyl and R² is ethyl.

16. A compound of formula (II): 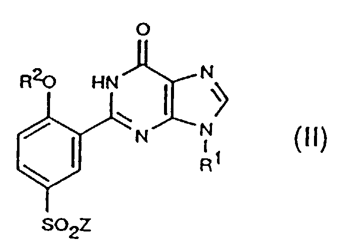 wherein Z is halo, and R¹ and R² are as previously defined in claim 7.

17. A compound according to claim 16 wherein Z is chloro.

## Patentansprüche

1. Verbindung der Formel (I): 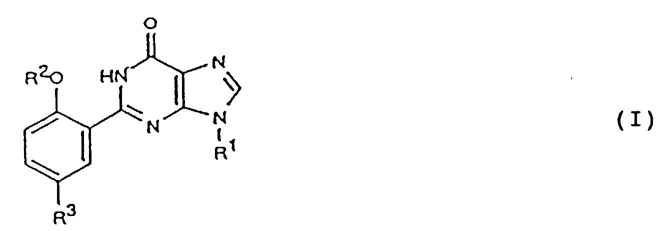 oder ein pharmazeutisch annehmbares Salz hievon, worin R¹ C₁-C₄-Alkyl bedeutet; R² C₂-C₄-Alkyl darstellt; R³ H oder SO₂NR⁴R⁵ ist; R⁴ und R⁵ zusammen mit dem Stickstoffatom, an das sie gebunden sind, eine Pyrrolidino-, Piperidino-, Morpholino-oder 4-N-(R₆)-1-Piperazinyl-Gruppe bilden; und R⁶ H oder C₁-C₃-Alkyl bedeutet.

2. Verbindung nach Anspruch 1, worin R¹ und R² jeweils unabhängig Ethyl- oder n-Propyl bedeuten; und R⁴ und R⁵ zusammen mit dem Stickstoffatom, an das sie gebunden sind, eine 4-N-(R₆)-1-Piperazinyl-Gruppe bilden.

3. Verbindung nach Anspruch 2, worin R¹ n-Propyl bedeutet; R² Ethyl darstellt; und R³ 1-Piperazinylsulfonyl oder 4-Methyl-1-piperazinylsulfonyl ist.

4. Pharmazeutische Zusammensetzung, welche eine Verbindung der Formel (I), oder ein pharmazeutisch annehmbares Salz hievon, nach einem der Ansprüche 1 bis 3, zusammen mit einem pharmazeutisch annehmbaren Verdünnungsmittel oder Träger umfaßt.

5. Verbindung der Formel (I), oder ein pharmazeutisch annehmbares Salz hievon, oder eine pharmazeutische Zusammensetzung, die eine der Einheiten enthält, nach einem der Ansprüche 1 bis 4, zur Verwendung in der Medizin.

6. Verwendung einer Verbindung der Formel (I), oder eines pharmazeutisch annehmbaren Salzes hievon, oder einer pharmazeutischen Zusammensetzung, die eine der Einheiten enthält, nach einem der Ansprüche 1 bis 4, bei der Herstellung eines Medikaments zur Behandlung von stabiler, instabiler und Variant-(Prinzmetal-) Angina, Hypertension, pulmonaler Hypertension, kongestivem Herzversagen, Atherosklerose, Schlaganfall, peripheren Gefäßerkrankungen, Zuständen einer verringerten Durchgängigkeit von Blutgefäßen, chronischem Asthma, Bronchitis, allergischem Asthma, allergischer Rhinitis, Glaukom oder durch Störungen der Darmmotilität charakterisierten Erkrankungen.

7. Verfahren zur Herstellung einer Verbindung der Formel (I): 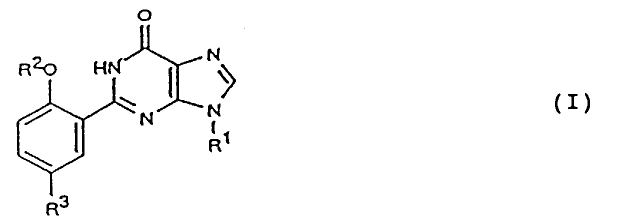 oder eines pharmazeutisch annehmbaren Salzes hievon, worin R¹ C₁-C₄-Alkyl bedeutet; R² C₂-C₄-Alkyl darstellt; R³ SO₂NR⁴R⁵ ist; R⁴ und R⁵ zusammen mit dem Stickstoffatom, an das sie gebunden sind, eine Pyrrolidino-, Piperidino-, Morpholino- oder 4-N-(R⁶)-1-Piperazinyl-Gruppe bilden; und R⁶ H oder C₁-C₃-Alkyl bedeutet; welches umfaßt: Umsetzen einer Verbindung der Formel (II): 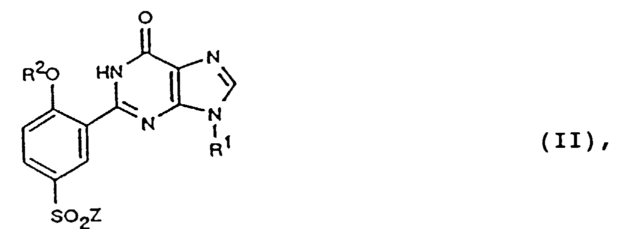 worin Z Halogen bedeutet, und R¹ und R² wie vorstehend in diesem Anspruch angegeben sind, mit einer Verbindung der Formel (III):
HNR⁴R⁵ (III),
worin R⁴ und R⁵ wie vorstehend in diesem Anspruch definiert sind, und gegebenenfalls Überführen des erforderlichen Produkts in ein pharmazeutisch annehmbares Salz hievon.

8. Verfahren nach Anspruch 7, worin ein 5-facher Überschuß einer Verbindung der Formel (III) zusammen mit einem C₁-C₃-Alkanol als Lösungsmittel verwendet wird.

9. Verfahren zur zur Herstellung einer Verbindung der Formel (I): 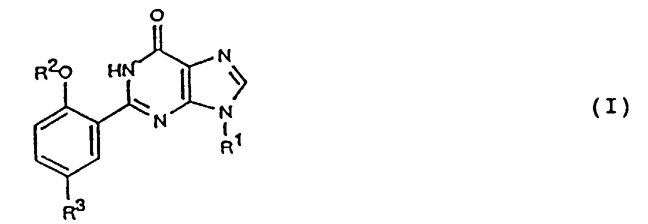 oder eines pharmazeutisch annehmbaren Salzes hievon, worin R¹ C₁-C₄-Alkyl bedeutet; R² C₂-C₄-Alkyl darstellt; und R³ H ist; welches umfaßt: Cyclisieren einer Verbindung der Formel (IV): 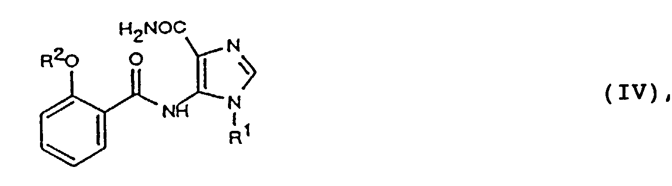 worin R¹ und R² wie vorstehend in diesem Anspruch definiert sind.

10. Verfahren nach Anspruch 9, bei welchem die Cyclisierung unter Verwendung einer Base, gegebenenfalls in Anwesenheit eines Wasserstoffperoxid-Überschusses, in wässerigem Ethanol erzielt wird.

11. Verfahren nach Anspruch 10, bei welchem die Base Natriumhydroxid oder Kaliumcarbonat ist.

12. Verfahren nach Anspruch 7 oder 8, wobei R¹ und R² jeweils unabhängig Ethyl oder n-Propyl bedeuten; und R⁴ und R⁵ zusammen mit dem Stickstoffatom, an das sie gebunden sind, eine 4-N-(R⁶)-1-Piperazinyl-Gruppe bilden.

13. Verfahren nach Anspruch 12, wobei R¹ n-Propyl bedeutet; R² Ethyl darstellt; und R³ 1-Piperazinylsulfonyl oder 4-Methyl-1-piperazinylsulfonyl ist.

14. Verfahren nach einem der Ansprüche 9 bis 11, wobei R¹ und R² jeweils unabhängig Ethyl oder n-Propyl bedeuten.

15. Verfahren nach Anspruch 14, wobei R¹ n-Propyl bedeutet, und R² Ethyl darstellt.

16. Verbindung der Formel (II): 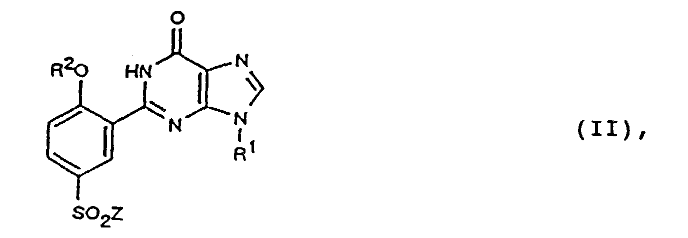 worin Z Halogen bedeutet, und R¹ und R² wie vorstehend in Anspruch 7 definiert sind.

17. Verbindung nach Anspruch 16, worin Z Chlor bedeutet.

## Revendications

1. Composé de formule (I) 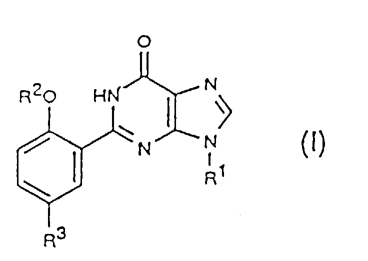 ou sel pharmaceutiquement acceptable d'un tel composé, dans lequel
R¹ représente alkyle en C₁-C₄ ;
R² représente alkyle en C₂-C₄ ;
R³ représente H ou SO₂NR⁴R⁵ ;
R⁴ et R⁵, pris ensemble avec l'atome d'azote auquel ils sont liés, forment un groupe pyrrolidino, pipéridino, morpholino ou 4-N- (R⁶) -1-pipérazinyle ; et
R⁶ représente H ou alkyle en C₁-C₃.

2. Composé selon la revendication 1, dans lequel R¹ et R² représentent chacun indépendamment éthyle ou n-propyle ; et R⁴ et R⁵, pris ensemble avec l'atome d'azote auquel ils sont liés, forment un groupe 4-N-(R⁶)-1-pipérazinyle.

3. Composé selon la revendication 2, dans lequel R¹ représente n-propyle ; R² représente éthyle ; et R³ représente 1-pipérazinylsulfonyle ou 4-méthyl-1-pipérazinylsulfonyle

4. Composition pharmaceutique comprenant un composé de formule (I), ou un sel pharmaceutiquement acceptable d'un tel composé, selon l'une quelconque des revendications 1 à 3, avec un diluant ou un support pharmaceutiquement acceptable.

5. Composé de formule (I), ou sel pharmaceutiquement acceptable d'un tel composé, ou composition pharmaceutique contenant ledit composé ou ledit sel, selon l'une quelconque des revendications 1 à 4, pour une utilisation en médecine.

6. Utilisation d'un composé de formule (I), ou d'un sel pharmaceutiquement acceptable d'un tel composé, ou d'une composition pharmaceutique contenant ledit composé ou ledit sel, selon l'une quelconque des revendications 1 à 4, pour la fabrication d'un médicament pour le traitement de l'angine de poitrine stable, instable et variable (Prinzmetal), l'hypertension, l'hypertension pulmonaire, les défaillances cardiaques congestives, l'athérosclérose, les attaques, la maladie vasculaire périphérique, les états de libre écoulement réduit des vaisseaux sanguins, l'asthme chronique, la bronchite, l'asthme allergique, la rhinite allergique, le glaucome ou des maladies caractérisées par des désordres de la motilité de l'intestin.

7. Procédé de préparation d'un composé de formule (I) : 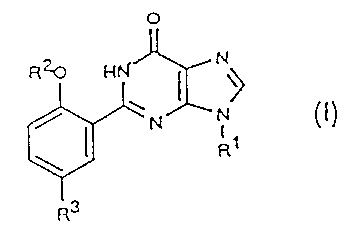 ou d'un sel pharmaceutiquement acceptable d'un tel composé, dans lequel :
R¹ représente alkyle en C₁-C₄ ;
R² représente alkyle en C₂-C₄ ;
R³ représente SO₂NR⁴R⁵ ;
R⁴ et R⁵, pris ensemble avec l'atome d'azote auquel ils sont liés, forment un groupe pyrrolidino, pipéridino, morpholino ou 4-N-(R⁶)-1-pipérazinyle; et
R⁶ représente H ou alkyle en C₁-C₃,
qui consiste à faire réagir un composé de formule (II) : 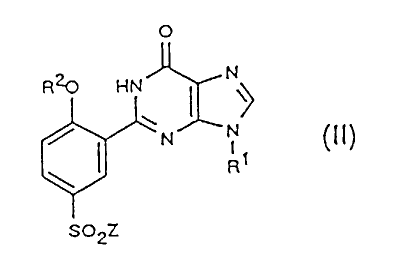 où Z représente halogéno, et R¹ et R² sont tels que précédemment définis dans cette revendication, avec un composé de formule (III) :
HNR⁴R⁵ (III)
dans laquelle R⁴ et R⁵ sont tels que précédemment définis dans cette revendication, et à transformer éventuellement le produit voulu en un sel pharmaceutiquement acceptable correspondant.

8. Procédé selon la revendication 7, dans lequel on emploie une quantité de 5 fois en excès d'un composé de formule (III) avec un alcanol en C₁-C₃ comme solvant.

9. Procédé de préparation d'un composé de formule (I) : 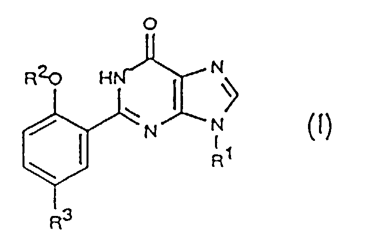 ou d'un sel pharmaceutiquement acceptable d'un tel composé, dans lequel
R¹ représente alkyle en C₁-C₄ ;
R² représente alkyle en C₂-C₄ ; et
R³ représente H ;
qui comporte la cyclisation d'un composé de formule (IV) : 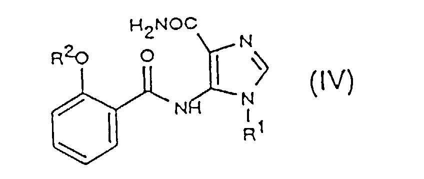 dans laquelle R¹ et R² sont tels que précédemment définis dans cette revendication.

10. Procédé selon la revendication 9, dans lequel la cyclisation est mise en oeuvre en utilisant une base, éventuellement en présence d'un excès de peroxyde d'hydrogène, dans de l'éthanol aqueux.

11. Procédé selon la revendication 10, dans lequel la base est l'hydroxyde de sodium ou le carbonate de potassium.

12. Procédé la revendication 7 ou la revendication 8, dans lequel R¹ et R² représentent chacun, indépendamment, éthyle ou n-propyle ; et R⁴ et R⁵, pris ensemble avec l'atome d'azote auquel ils sont liés, forment un groupe 4-N-(R⁶)-1-pipérazinyle.

13. Procédé selon la revendication 12, dans lequel R¹ représente n-propyle ; R² représente éthyle et R³ représente 1-pipérazinylsulfonyle ou 4-méthyl-1-pipérazinylsulfonyle.

14. Procédé selon l'une quelconque des revendications 9 à 11, dans lequel R¹ et R² représentent chacun, indépendamment, éthyle ou n-propyle.

15. Procédé selon la revendication 14, dans lequel R¹ représente n-propyle et R² représente éthyle.

16. Composé de formule (II) 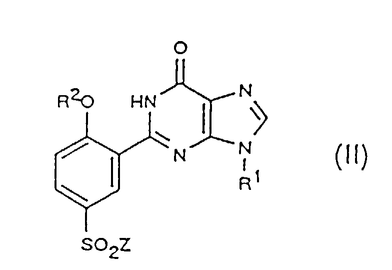 dans lequel Z représente halogéno, et R¹ et R² sont tels que précédemment définis dans la revendication 7.

17. Composé selon la revendication 16, dans lequel Z représente chloro.
